# EUROPEAN PATENT SPECIFICATION

(11) **EP 2 769 226 B1**
(45) Date of publication and mention of the grant of the patent: **24.05.2017**
(21) Application number: 12747947.5
(22) Date of filing: 12.07.2012
(51) Int. Cl.: G01N 33/58, G01N 21/64, G01N 33/66

(54) **COMBINATIONS OF FLUORPHORES AND PYRIDINIUM BORONIC ACID QUENCHERS FOR USE IN ANALYTE SENSORS**
KOMBINATIONEN AUS FLUORPHOREN UND PYRIDINBORSÄURE-LÖSCHMITTELN ZUR VERWENDUNG IN ANALYTSENSOREN
COMBINAISONS DE FLUOROPHORES ET DE DÉSACTIVATEURS DE PYRIDINIUM-ACIDE BORONIQUE DESTINÉES À ÊTRE UTILISÉES DANS DES CAPTEURS D'ANALYTES

(30) Priority: 15.07.2011 US 201161508509 P
(43) Date of publication of application: 27.08.2014
(73) Proprietor: MEDTRONIC MINIMED, INC., Northridge, CA 91325-1219 (US)
(72) Inventor: WESSLING, Ritchie, A., Watsonville, CA 95076 (US); SURI, Jeff, T., Rancho Santa Margarita, CA 92688 (US); GAMSEY, Soya, Huntington Beach, CA 92647 (US)
(74) Representative: Ruschke, Hans Edvard
(86) International application number: PCT/US2012/046513
(87) International publication number: WO 2013/012687

(56) References cited:
- US-A1- 2002 193 672
- US-A1- 2008 305 009
- US-A1- 2009 061 528
- US-A1- 2009 081 803
- GUNTER ZENKL ET AL: "Sugar-Responsive Fluorescent Nanospheres", MACROMOLECULAR BIOSCIENCE, vol. 8, no. 2, 11 February 2008 (2008-02-11), pages 146-152, XP055046244, ISSN: 1616-5187, DOI: 10.1002/mabi.200700174
- HUA YANG ET AL: "Scalable Synthesis of Lissamine Rhodamine B Sulfonyl Chloride and Incorporation of Xanthene Derivatives onto Polymer Supports", SYNTHESIS, vol. 2008, no. 6, 1 March 2008 (2008-03-01), pages 957-961, XP055034973, ISSN: 0039-7881, DOI: 10.1055/s-2008-1032172
- CORDES DAVID B: "Optical glucose detection across the visible spectrum using anionic fluorescent dyes and a viologen quencher in a two-component saccharide sensing system", ORGANIC & BIOMOLECULAR CHEMISTRY, ROYAL SOCIETY OF CHEMISTRY, GB, vol. 3, no. 9, 7 May 2005 (2005-05-07), pages 1708-1713, XP008081119, ISSN: 1477-0520, DOI: 10.1039/B418953A
- DI WANG ET AL: "Stimuli-Responsive Fluorescent Poly( N -isopropylacrylamide) Microgels Labeled with Phenylboronic Acid Moieties as Multifunctional Ratiometric Probes for Glucose and Temperatures", MACROMOLECULES, vol. 44, no. 7, 12 April 2011 (2011-04-12) , pages 2282-2290, XP055046889, ISSN: 0024-9297, DOI: 10.1021/ma200053a

## Description

### BACKGROUND OF THE INVENTION

### Field of the Invention

Aspects of the invention relate generally to the detection of polyhydroxyl-substituted organic molecules, and in particular to the use of fluorescent dyes combined with pyridinium salts functionalized with boronic acids as quenchers for use in glucose sensors.

### Description of the Related Art

Fluorescent dyes and analyte-binding quenchers that modulate fluorescence of the dye upon binding an analyte are known and have been used in two-component sensing systems for analyte detection. See e.g., US Patent Nos. 6,653,141, 6,627,177, and 7,470,420; each of which is incorporated herein in its entirety by reference thereto. These systems employ a wide range of fluorescent dyes and boronic acid-appended viologens as analyte binding quenchers.

The use of analyte-binding pyridinium boronic acid quenchers preferably in combination with pyrene sulfonate-based dyes to make sensors for the detection of glucose is described in US 7,939,664.

Investigators have made fluorophores with N-benzyl-2-boronic acid pyridinium groups attached to a porphyrin ring (Arimori, S. et al. 1996 J Am Chem Soc 118:245-246). They were used to promote aggregation with another porphyrin substituted with saccharide groups via intermolecular ester formation. Benzyl-2- and benzyl-4-boronic acid substituents on the pyridine nitrogens in substituted porphyrins were also described (Arimori et al. 1996 Chemistry Letters 25:77). They were used to distinguish chiral orientation in sugars. It was shown that the fluorescence was reduced by complex formation between these porphyrins and anthraquinone disulfonates. The complex was dissociated by reaction of the boronic acids with fructose resulting in an increase in fluorescence. The quenching moiety in this case was the anthraquinone component (Arimori et al. 1995 J Chem Soc, Chem Commun 9:961-962). Subsequently, investigators described a dye with a pyridine ring in the structure, substituted on the nitrogen with a benzyl-2-boronic acid group (Takeuchi et al, 1996 Bull Chem Soc (Jpn) 69:2613-2618). It was noted that the pyridinium group in ortho- position enhances reactivity of boronic acids with diols. This dye was used to detect nucleotides. In a paper concerning trialkyl ammonium substituted benzyl-2-boronic acids, a generic formula for N-benzyl-2-boronic acid derivatives of para-substituted pyridines was given, where the substituent was specified as an R-group (*i.e.,* alkyl) (Takeuchi et al. 1996 Tetrahedron 52:12931-12940).

A pyridinium salt without a boronic acid substituent was used as a reference compound in a quenching study (Cordes et al. 2005 Langmuir 21:6540-6547). Other investigators measured the fluorescence quenching activity and glucose response of the three isomers of N-benzylboronic acid pyridinium salts. These compounds showed poor quenching of pyranine fluorescence and gave no glucose response (See e.g., "Detection of glucose with arylboronic acid containing viologens and fluorescent dyes: Progress toward a continuous glucose monitoring system for in vivo applications" Cappuccio, Frank E., Ph.D. Dissertation; UNIVERSITY OF CALIFORNIA, SANTA CRUZ, 2004).

A comparative study was reported on the quenching of Ru(bpy)₃ by methyl viologen (MV) and a series of 4-substituted N-methyl pyridiniums (Jones and Malba 1985 J Org Chem 50:5776-5782). This study showed that pyridiniums substituted in the 4- position with electron withdrawing groups conjugated to the ring behaved like MV. These compounds showed reversible reduction at similar potentials and had Stern-Volmer (S-V) constants in the same range.

Alkyl pyridinium surfactants have been widely studied as fluorescence quenchers. Fluorophores that have been successfully quenched include polycyclic aromatic hydrocarbons (Pandey et al. 1999 Talanta 48:1103-1110; Palit et al. 1997 Chem Phys Lett 269:286-292; Wadek and Tucker 2000 Talanta 53:571-578; Mao et al. 2003 J Sep Sci 26:1643-1649), aminofluorene (Saha et al. 1999 J Photochem Photobiol A 121:191-198), and carbazole substituents on polymers (Yatsue et al. 1992 J Phys Chem 96:10125-10129).

Most studies were with simple N-alkyl pyridinium salts where the alkyl group was large enough to make the salt surface active. The polymer study was carried out with para-substituted N-alkyl pyridiniums, including derivatives of 4-acetyl pyridine, methyl isonicotinate, and isonicotinamide. In other studies with ring substituted pyridiniums, bis-picolinium salts with N,N'-alkylene bridging groups were used to quench the fluorescence of naphthols. The quenching efficiency of the bis compounds was substantially higher than that of a mono-picolinium control; and was highest for the compound with a methylene linker. (Panda et al. 1998 J Photochem Photobio A 113:73-80).

Fluorescent dyes, including 8-hydroxypyrene-1,3,6-trisulfonic acid (HPTS) and its derivatives, are known and have been used in analyte detection. See *e.g.,* U.S. Patent Nos. 6,653,141, 6,627,177, 5,512,246, 5,137,833, 6,800,451, 6,794,195, 6,804,544, 6,002,954, 6,319,540, 6,766,183, 5,503,770, 5,763,238, 7,317,111, 7,667,048, 7,417,164, 7,751,863, 7,824,918, 7,939,664, 7,829,341 and 7,470,420 and U.S. Publication No. 2004/0028612 A1; each of which are incorporated by reference in their entireties.

Other fluorescent dyes that have been used in saccharide sensing include fluorescein, Lucifer yellow, sulforhodamine B, sulforhodamine 101, carboxytetramethylrhodamine, tetrakis(4-sulfophenyl)porphine, tetrakis(4-carboxyphenyl)porphine, potassium perylenetetracarboxylate.^{ref} Some of these dyes are not amenable to immobilization and are not suitable for *in vivo* glucose detection.

There accordingly remains a need in this field for new fluorophores, made by environmentally acceptable processes, capable of being be immobilized in a polymer.

### SUMMARY OF THE INVENTION

One embodiment is an anionic, non-pyrene sulfonate-based fluorescent dye that is excited by visible light and emits at a wavelength greater than 500 nm, preferably at or greater than 510 nm, 520 nm, 530 nm, 540 nm or 550 nm, wherein the dye is functionalized with at least one reactive group.

In some embodiments, the dye is excited by blue light having a wavelength of from 435-500 nm.

The reactive group comprises an anionic group and an ethylenically unsaturated polymerizable group.

The polymerizable group comprises aminoCysMA.

In some embodiments, the anionic group is selected from the group consisting of sulfonic acids, phosphonic acids, carboxylic acids, phenols, and their salts.

In some embodiments, the anionic, non-pyrene sulfonate-based fluorescent dye is derived from the group consisting of sulforhodamine B, sulforhodamine 101, carboxytetramethylrhodamine, tetrakis(4-sulfophenyl)porphine, tetrakis(4-carboxyphenyl)porphine, potassium perylenetetracarboxylate, Alexa Fluor 532, Alexa Fluor 546, Alexa Fluor 568, Alexa Fluor 594, BODIPY 576/589, BODIPY 581/591, BODIPY TR-X, SNARF-1, and Texas Red.

Some embodiments are dyes according to the following formula:
Dyeⁿ-R, wherein 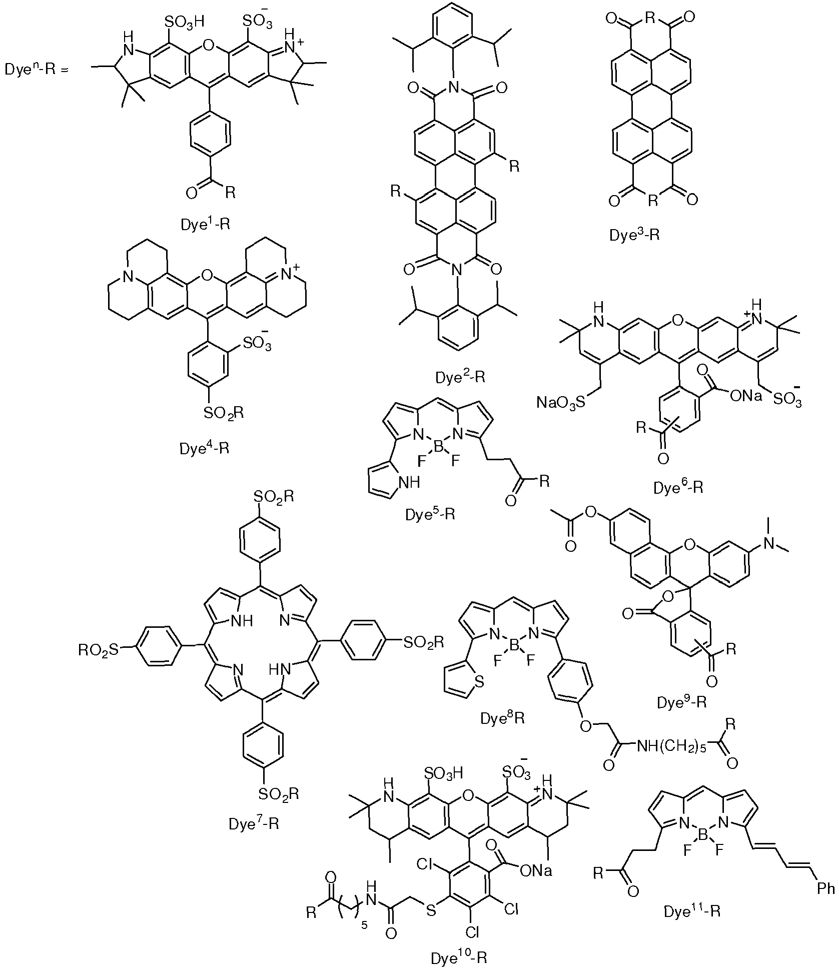 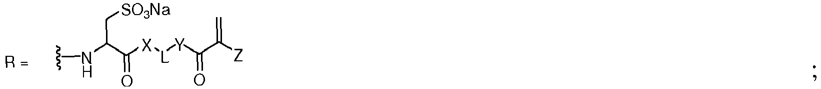
   X is NH, Y is NH L is -(CH₂)₃- or and Z is H or CH3.

Other embodiments relate to a method of making a dye comprising the step of: 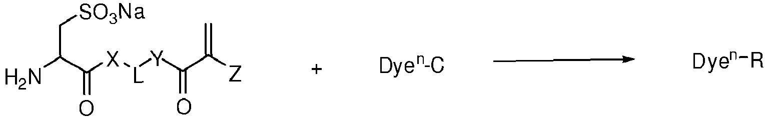 wherein: 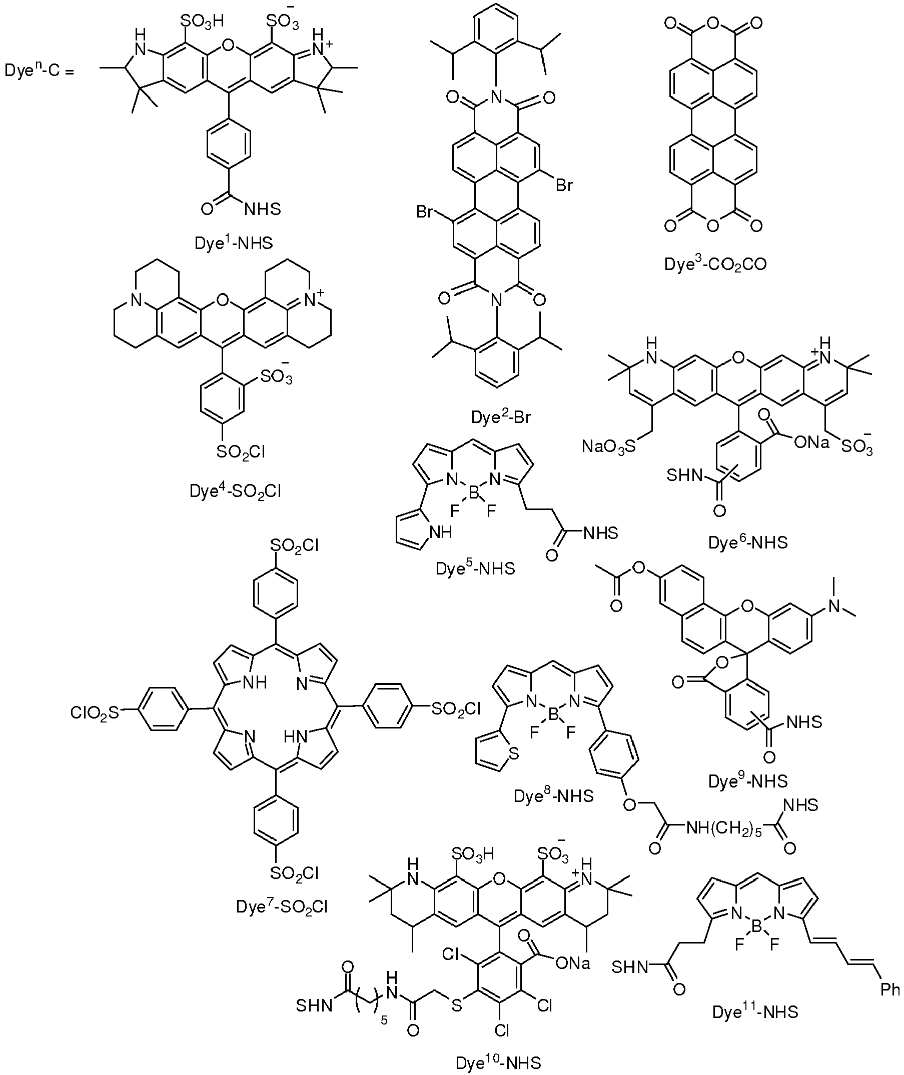 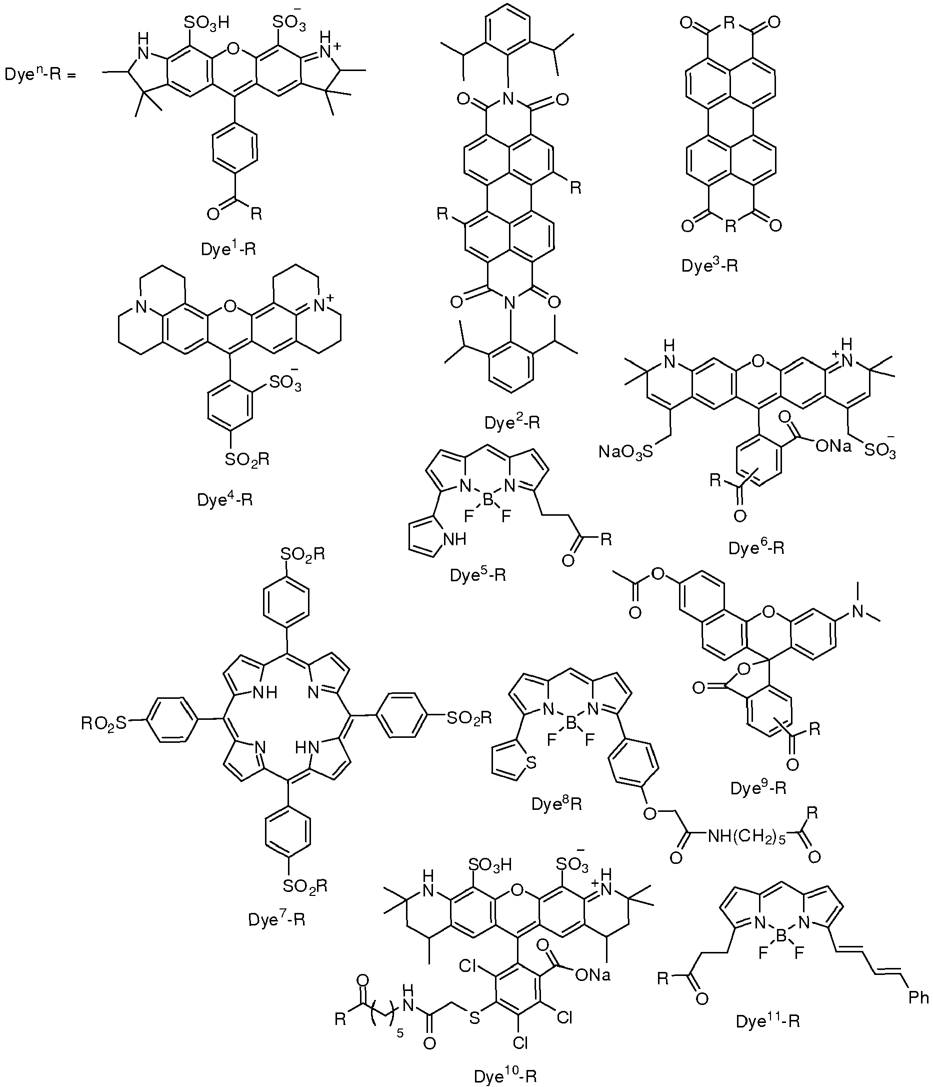 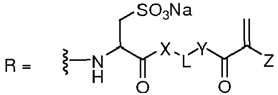
X is NH, Y is NH, L is -(CH₂)₃ - and and Z is H or CH₃.

Another embodiment disclosed herein is the compound:

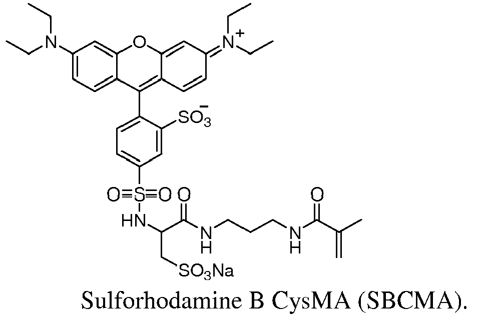

Also disclosed herein is a method of making Sulforhodamine B CysMA (SBCMA), comprising the step of:

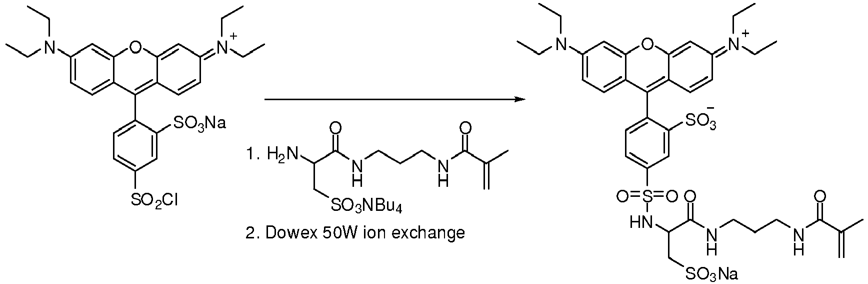

Another embodiment is an analyte sensor for detecting a polyhydroxyl compound comprising an anionic, non-pyrene sulfonate-based fluorescent dye that is excited by visible light and emits at a wavelength greater than 500 nm, preferably at or greater than 510 nm, 520 nm, 530 nm, 540 nm or 550 nm, wherein the dye is functionalized with at least one reactive group; and a pyridinium boronic acid quencher wherein dye and quencher are coupled to a polymer.

In some embodiments, the quencher comprises a monovalent, boronic 1. acid-substituted pyridinium group.

In other embodiments, the quencher comprises a boronic acid-substituted polypyridinium, wherein said polypyridinium comprises two or more boronic acid-appended pyridinium groups covalently coupled by a non-conjugated linker.

Also disclosed herein is a device for determining an analyte concentration comprising an analyte sensor for detecting a polyhydroxyl compound comprising an anionic, non-pyrene sulfonate-based fluorescent dye that is excited by visible light and emits at a wavelength greater than 500 nm, preferably at or greater than 510 nm, 520 nm, 530 nm, 540 nm or 550 nm, wherein the dye is functionalized with at least one reactive group; and a pyridinium boronic acid quencher wherein dye and quencher are coupled to a polymer.

### BRIEF DESCRIPTION OF THE DRAWINGS

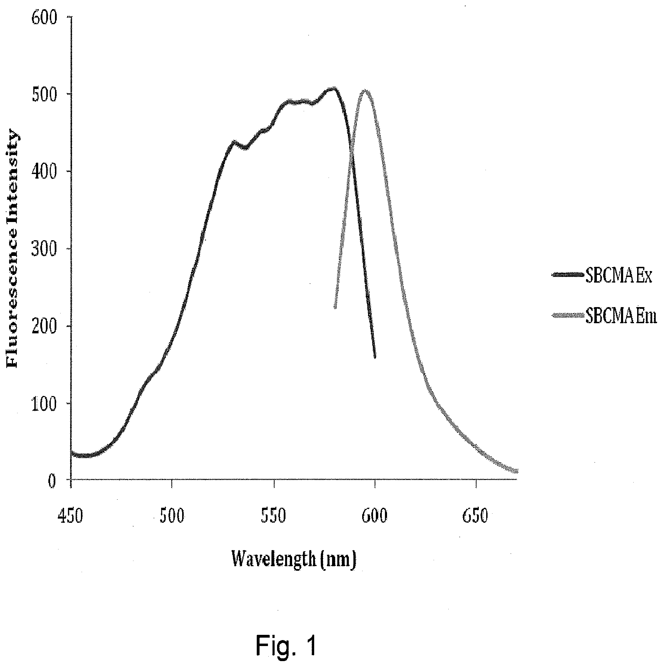

FIG. 1 shows the excitation and emission spectra of Sulforhodamine B CysMA (SBCMA) in pH 7.4 PBS.

### DETAILED DESCRIPTION OF THE PREFERRED EMBODIMENT

Analyte sensors comprising fluorescent dyes and analyte-binding quenchers that modulate the fluorescence of the dye upon binding an analyte are known and have been used to measure analyte concentrations, especially glucose concentration in body fluids. Most sensing systems employ dyes to which the analyte receptor is covalently bonded. A two-component approach offers advantages over such systems.

Although Applicants do not intend to be bound by the proposed mechanism of action, one mechanism that may be employed in some of the preferred indicator systems is the formation of a ground state complex between the analyte-binding moiety and the fluorescent dye. As a result of the formation of the complex, the fluorescence may be quenched. When the boronic acid group on the preferred analyte-binding moiety reacts with a polyhydroxyl-substituted organic molecule such as glucose, the boron becomes negatively charged. This weakens the complex, resulting in dissociation, and an increase in fluorescence that is related to glucose concentration.

The sensing systems disclosed herein for the detection of polyhydroxyl-substituted organic molecules (*e.g*., sugars) are made from novel reactive fluorescent dyes. In some embodiments, the fluorescent dyes exhibit the following characteristics. They are: 1) compatible with aqueous media; 2) excited by visible light, preferably blue light, and emit at wavelengths at or greater than 500 nm, preferably at or greater than 510 nm, 520 nm, 530 nm, 540 nm or 550 nm; 3) negatively charged preferably with at least two anionic groups; 4) susceptible to quenching by a pyridinium boronic acid quencher; and 5) amenable to immobilization.

The sensing systems disclosed herein for the detection of polyhydroxyl-substituted organic molecules (*e.g.*, sugars) further comprise the class of pyridinium salts functionalized with boronic acids as the analyte binding moieties. In some embodiments, the analyte-binding pyridinium quenchers exhibit at least some of the following characteristics. They are: 1) compatible with aqueous media; 2) good electron acceptors and the electron transfer process is reversible; 3) capable of quenching the fluorescence of dyes emitting at or greater than 550 nm 4) substituted with boronic acid groups; 5) positively charged, preferably with at least one cationic group per boronic acid; and 6) amenable to immobilization.

As used herein, "boronic acid" refers to a structure -B(OH)₂. It is recognized by those skilled in the art that a boronic acid may be present as a boronate ester at various stages in the synthesis of the quenchers. Boronic acid is meant to include such esters.

"Fluorophore" refers to a substance that when illuminated by light at a particular wavelength emits light at a longer wavelength; *i.e.,* it fluoresces.

"Quencher" refers to a compound that reduces the emission of a fluorophore when in its presence.

"Viologen" refers generally to compounds having the basic structure of a nitrogen containing conjugated N-substituted heterocyclic aromatic bis-onium salt, such as 2,2'-, 3,3'- or 4,4'-N,N' bis-(benzyl) bipyridium dihalide (*i.e.,* dichloride, bromide chloride), etc. Viologen also includes the substituted phenanthroline compounds.

"Pyridinium" refers to a pyridine substituted on the nitrogen to form a positively charged onium salt, optionally substituted at other positions on the pyridine ring.

"Reactive group" refers to substituents used to covalently bind the sensing moieties to a substrate.

### Fluorescent Dyes

Anionic fluorescent dyes that are excited by visible light and emit at wavelengths at or greater than 500 nm, preferably at or greater than 510 nm, 520 nm, 530 nm, 540 nm or 550 nm, that are functionalized with reactive groups including both polymerizable groups or coupling groups are disclosed. These compounds are structurally configured and functionally adapted to interact with analyte binding quenchers and may be used in glucose sensors as alternatives to pyrene sulfonate derivatives, such HPTS-CysMA described e.g., in US Patents 7,417,164 and 7,939,664. Preferably the dyes are resistant to photodegradation. Preferably, the dyes are excited by blue light and emit at wavelengths equal to or greater than 500 nm, preferably at or greater than 510 nm, 520 nm, 530 nm, 540 nm or 550 nm. A large Stokes shift and long wavelength emission allow for greater accuracy in measuring emission intensity with less background interference. Preferably the dyes are made by environmentally benign processes.

In one embodiment, the dyes are synthesized by coupling a dye substituted with a reactive group with a multifunctional molecule comprising an anionic group substituted core structure further substituted with two selectively reactive functional groups. The differential reactivity allows the core structure to be sequentially coupled both to the reactive dye intermediate and to a third reactive group which is preferably an ethylenically unsaturated polymerizable group. Exemplary of this class of multifunctional molecules is cysteic acid.

In one embodiment, the polymerizable group is aminoCysMA. Polymerizable groups may be ethylenically unsaturated groups including acryoyl, methacryloyl, acrylamido, methacrylamido, styryl, and the like. Coupling groups used to bond the dye to an existing polymer or substrate include, but are not limited to, sulfonyl chlorides, carboxylic acids, aldehydes, alkynes and azides, as well as activated esters, such as succinimides and nitrobenzoates.

Anionic groups include sulfonic acids, phosphonic acids, carboxylic acids, phenols, and their salts. Interaction is further enhanced by the presence of acid groups that are fully ionized at physiological pH. Therefore, in some embodiments, sulfonate ions are preferred.

Anionic fluorescent dyes with emission maxima at or greater than 500 nm, preferably at or greater than 510 nm, 520 nm, 530 nm, 540 nm or 550 nm, useful as reporters in two-component analyte sensing systems are disclosed in accordance with some embodiments.

Useful dyes include conventional dyes substituted with a preactive group. They include derivatives of sulforhodamine B, sulforhodamine 101, carboxytetramethylrhodamine, tetrakis(4-sulfophenyl)porphine, tetrakis(4-carboxyphenyl)porphine, potassium perylenetetracarboxylate, Alexa Fluor 532, Alexa Fluor 546, Alexa Fluor 568, Alexa Fluor 594, BODIPY 576/589, BODIPY 581/591, BODIPY TR-X, SNARF-1, and Texas Red.

Methods for making the anionic fluorescent dyes functionalized with polymerizable groups are disclosed in accordance with some embodiments.

A method is disclosed in which the polymerizable group is coupled to cysteic acid by amidation or esterification to yield a multifunctional molecule for subsequent attachment to a reactive dye substrate 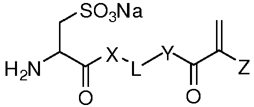 where X is NH or O, Y is NH or O and L is a divalent linker selected from the group consisting of a direct bond and a lower alkylene having 1 to 8 carbon atoms, optionally terminated with or interrupted by one or more divalent connecting groups selected from the group consisting of sulfonamide (-SO2NH-), amide -(C=O)N-, ester -(C=O)-O-, ether -O-, sulfide -S-, sulfone (-SO2-), phenylene -C6H4-, urethane -NH(C=O)-O-, urea -NH(C=O)NH-, thiourea -NH(C=S)-NH-, amide -(C=O)NH-, amine -NR- (where R is defined as alkyl having 1 to 6 carbon atoms) or combinations thereof; Z is H or CH₃. Preferably L is an alkylene of 2 to 6 carbons or an oxyethylene (-O-CH₂CH₂-)ₙ where n is 1 to 4.

Composition comprising the adduct of reactive derivatives of sulforhodamine B, sulforhodamine 101, carboxytetramethylrhodamine, tetrakis(4-sulfophenyl)porphine, tetrakis(4-carboxyphenyl)porphine, potassium perylenetetracarboxylate, Alexa Fluor 532, Alexa Fluor 546, Alexa Fluor 568, Alexa Fluor 594, BODIPY 576/589, BODIPY 581/591, BODIPY TR-X, SNARF-1, Texas Red with the polymerizable group derived from cysteic acid are disclosed.

### Analyte Sensors

Analyte sensors comprising a fluorescent dye with an emission wavelength at or greater than 500 nm, preferably at or greater than 510 nm, 520 nm, 530 nm, 540 nm or 550 nm, and a pyridinium boronic acid quencher are disclosed in accordance with some embodiments.

The sensing systems used in accordance with some embodiments comprise a fluorophore operably coupled to an analyte binding moiety, wherein analyte binding causes an apparent optical change in the fluorophore concentration (e.g., emission intensity). For example, a glucose binding moiety, e.g., quencher P-1 as described in US 7,939,664, that is operably coupled to a fluorescent dye, will quench the emission intensity of the dye, wherein the extent of quenching is reduced upon glucose binding resulting in an increase in emission intensity related to glucose concentration. In certain embodiments, the sensing systems comprise a dye having at least two anionic groups and a pyridinium quencher having at least one boronic acid. In certain embodiments, the anionic groups are sulfonic acids or salts thereof. In certain embodiments, the quencher moiety comprises at least two pyridinium boronic acid groups covalently coupled by a non-conjugated linker. In certain preferred embodiments, the spacing of the pyridinium groups allows cooperative binding of two boronic acid groups with a single glucose molecule to enhance selectivity. In further embodiments, the sensing systems may also comprise a means for immobilizing the sensing moieties (e.g., dye-quencher) such that they remain physically close enough to one another to interact (quenching). Where *in vivo* sensing is desired, such immobilizing means are preferably insoluble in an aqueous environment (e.g., intravascular), permeable to the target analytes, and impermeable to the sensing moieties. Typically, the immobilizing means comprises a water-insoluble organic polymer matrix. For example, the dye and quencher may be effectively immobilized within a DMAA (*N, N-*dimethylacrylamide) hydrogel matrix by copolymerization with the hydrogel forming monomers (described in detail below), which allows glucose sensing *in vivo.*

Some exemplary fluorophores and immobilizing means and sensing systems employing said fluorophores are set forth in greater detail below. In some embodiments, useful dyes are prepared by coupling aminoCysMA to a reactive dye through a coupling group (Figure 2) and copolymerizing the dye monomer thus formed with hydrogel forming monomers. The reactive dyes include but are not limited to derivatives of sulforhodamine B, sulforhodamine 101, carboxytetramethylrhodamine, tetrakis(4-sulfophenyl)porphine, tetrakis(4-carboxyphenyl)porphine, potassium perylenetetracarboxylate, Alexa Fluor 532, Alexa Fluor 546, Alexa Fluor 568, Alexa Fluor 594, BODIPY 576/589, BODIPY 581/591, BODIPY TR-X, SNARF-1, and Texas Red. The coupling groups used to bond the dye to a substrate include, but are not limited to, sulfonyl chlorides, carboxylic acids, aldehydes, alkynes and azides, as well as activated esters, such as succinimides and nitrobenzoates.

### Methods of Making Dyes

The following general method may be used to make the monomeric dyes disclosed herein: 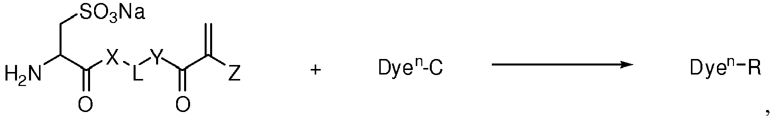 wherein: 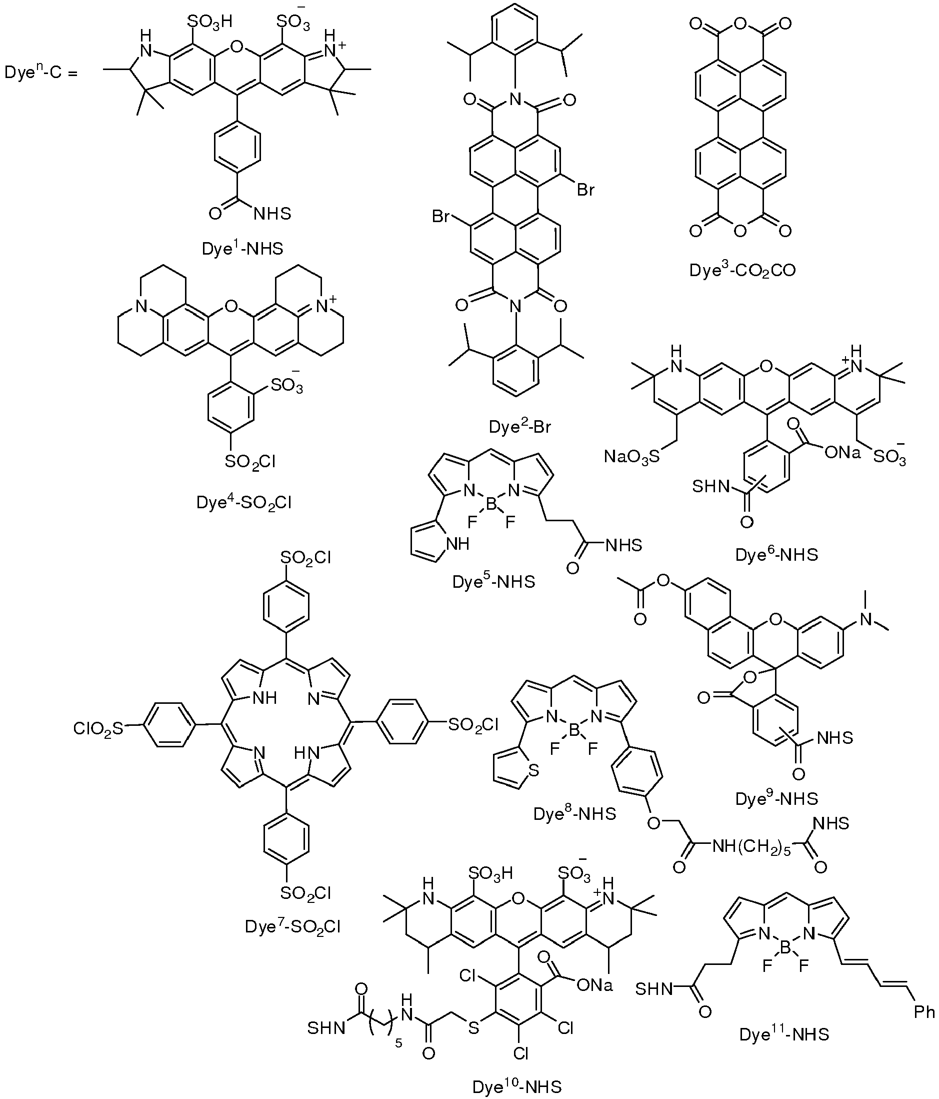 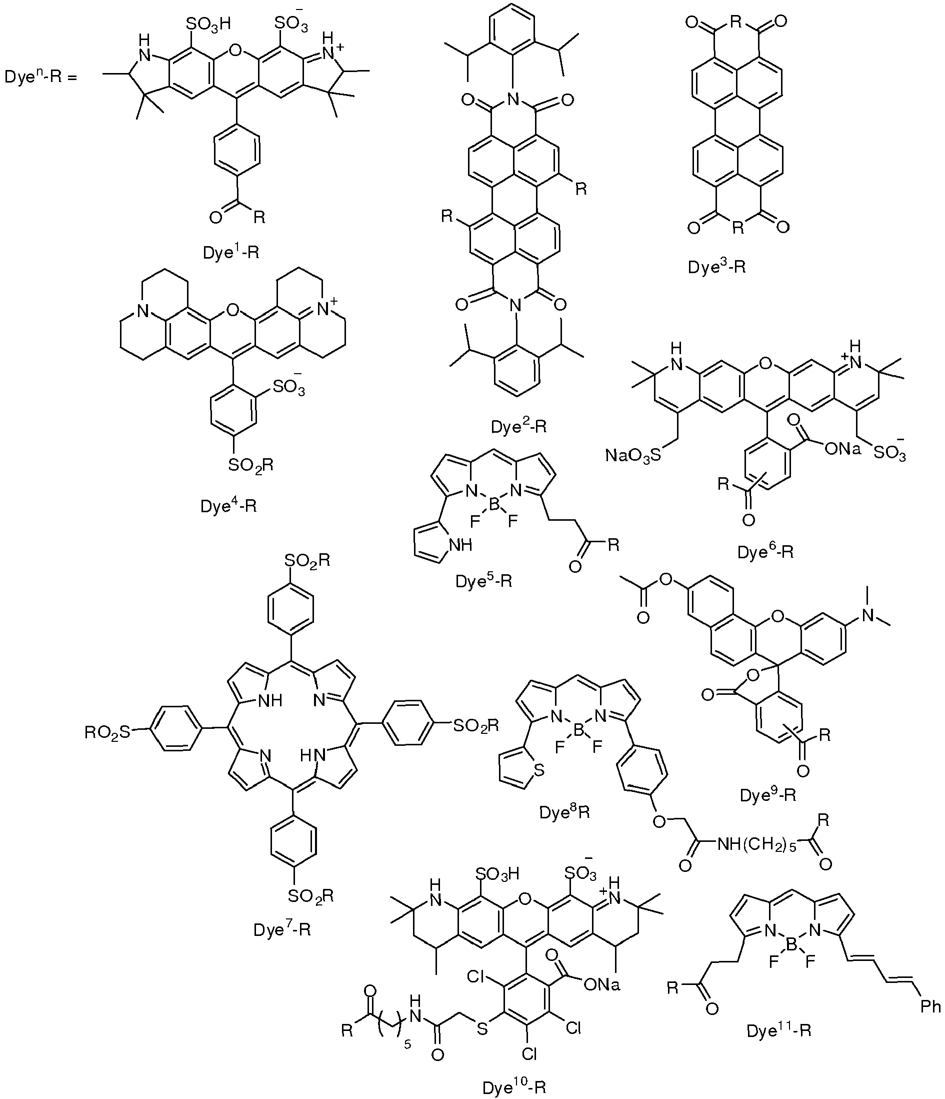 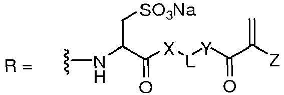
where:
   Dyeⁿ-C is a reactive dye;
   C is a coupling group;
   X is NH;
   Y is NH;
   L is -(CH₂)₃- and Z is H or CH₃.

In one embodiment, the fluorescent dye may be a pendant group bonded to a polymer through a divalent linking group.

In one embodiment, the fluorescent dye may be a unit in a polymer chain. For example, polymer matrices comprising a fluorescent dye immobilized as a unit in a polymer chain are discussed below.

In other variations, the sulfonic acid groups may be replaced with other anionic groups; e.g., phosphoric, carboxylic, etc.

For *in vivo* applications, the sensor is used in a moving stream of physiological fluid which contains one or more polyhydroxyl organic compounds or is implanted in tissue such as muscle which contains said compounds. Therefore, it is preferred that none of the sensing moieties escape from the sensor assembly. Thus, for use in vivo, the sensing components are preferably part of an organic polymer sensing assembly.

### Polymer Matrices for Sensors

In some embodiments, the dye is bound to a polymer through sulfonamide functional groups. The polymeric dyes may be water soluble, water insoluble, organic-solvent soluble or organic-solvent insoluble. For sensing to occur, the sensing moieties (analyte, dye, and quencher) are in close physical proximity to allow interaction, *i.e.,* mixed on a molecular level and in equilibrium with the species to be detected for quenching to occur.

Preferably, the sensing moieties are immobilized in an insoluble polymer matrix, which is freely permeable to glucose. The polymer matrix is comprised of organic, inorganic or combinations of polymers thereof. The matrix may be composed of biocompatible materials. Alternatively, the matrix is coated with a second biocompatible polymer that is permeable to the analytes of interest.

The function of the polymer matrix is to hold together and immobilize the fluorophore and quencher moieties while at the same time allowing contact with the analyte, and binding of the analyte to the boronic acid. To achieve this effect, the matrix is preferably insoluble in the medium, and in close association with it by establishing a high surface area interface between matrix and analyte solution. For example, an ultra-thin film or microporous support matrix is used. Alternatively, the matrix is swellable in the analyte solution, e.g. a hydrogel matrix is used for aqueous systems. In some instances, the sensing polymers are bonded to a surface such as the surface of a light conduit, or impregnated in a microporous membrane. In all cases, the matrix preferably does not interfere with transport of the analyte to the binding sites so that equilibrium can be established between the two phases. Techniques for preparing ultra-thin films, microporous polymers, microporous sol-gels, and hydrogels are established in the art. All useful matrices are defined as being analyte permeable.

Hydrogel polymers are used in some embodiments. The term, hydrogel, as used herein refers to a polymer that swells substantially, but does not dissolve in water. Such hydrogels may be linear, branched, or network polymers, or polyelectrolyte complexes, with the proviso that they contain no soluble or leachable fractions. Typically, hydrogel networks are prepared by a crosslinking step, which is performed on watersoluble polymers so that they swell but do not dissolve in aqueous media. Alternatively, the hydrogel polymers are prepared by copolymerizing a mixture of hydrophilic and crosslinking monomers to obtain a water swellable network polymer. Such polymers are formed either by addition or condensation polymerization, or by combination process. In these cases, the sensing moieties are incorporated into the polymer by copolymerization using monomeric derivatives in combination with network-forming monomers. Alternatively, reactive moieties are coupled to an already prepared matrix using a post polymerization reaction. Said sensing moieties are units in the polymer chain or pendant groups attached to the chain.

The hydrogels that may be useful are also monolithic polymers, such as a single network to which both dye and quencher are covalently bonded, or multi-component hydrogels. Multi-component hydrogels include interpenetrating networks, polyelectrolyte complexes, and various other blends of two or more polymers to obtain a water swellable composite, which includes dispersions of a second polymer in a hydrogel matrix and alternating microlayer assemblies.

Monolithic hydrogels are typically formed by free radical copolymerization of a mixture of hydrophilic monomers, including but not limited to hydroxyethyl methacrylate, polyethylene glycol monomethacrylate, methacrylic acid, hydroxyethyl acrylate, N-vinyl pyrrolidone, acrylamide, *N,N*'-dimethyl acrylamide, and the like; ionic monomers include methacryloylaminopropyl trimethylammonium chloride, diallyl dimethyl ammonium chloride, vinyl benzyl trimethyl ammonium chloride, sodium sulfopropyl methacrylate, 2,2'-acrylamido methyl propane sulfonic acid, and the like; crosslinkers include ethylene dimethacrylate, polyethylene glycol dimethacrylate, trimethylolpropane triacrylate, *N,N'*-methylene bis acrylamide, and the like. The ratios of monomers are chosen to optimize network properties including permeability, swelling index, and gel strength using principles well established in the art. In one embodiment, the dye moiety is derived from an ethylenically unsaturated derivative of a dye molecule, such as sulforhodamine B CysMA the quencher moiety is derived from an ethylenically unsaturated pyridinium such as 4-(amidopropylmethacrylamide) N-benzyl (2-boronic acid) pyridinium bromide and the matrix is made from DMAA and *N,N*-methylenebisacrylamide. The concentration of dye is chosen to optimize emission intensity. The ratio of quencher to dye is adjusted to provide sufficient quenching to produce the desired measurable signal.

### Semi-Permeable Membranes

In other embodiments, soluble polymers comprising dye and quencher moieties can be confined by a semi-permeable membrane that allows passage of the analyte but blocks passage of the sensing moieties. This can be realized by using as sensing moieties soluble molecules that are substantially larger than the analyte molecules (molecular weight of at least twice that of the analyte or greater than 1000 preferably greater than 5000); and employing a selective semipermeable membrane such as a dialysis or an ultrafiltration membrane with a specific molecular weight cutoff between the two so that the sensing moieties are quantitatively retained.

### EXAMPLE

### Synthesis of sulforhodamine B CysMA

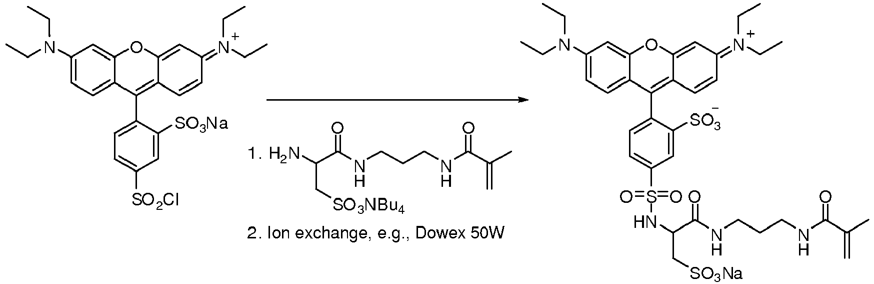

A 10-mL round bottom flask was charged with sulforhodamine B sulfonyl chloride (0.26 mmols, 150 mg), methylene chloride (2 ml), aminoCysMA (0.312 mmols, 166 mg) and triethyl amine (0.6 mmols, 61 mg) and stirred at room temperature for 15h. The purple solution was concentrated to dryness and purified by column chromatography using gradient elution (5 to 30 % MeOH in CH₂Cl₂). The purified fractions were combined and concentrated in vacuo and the residue was dissolved in water (10 mL). Dowex 50W (H+ form, 2 g) was added to the solution and the mixture was stirred for 10 minutes and filtered. The filtrate was treated with Dowex 50W (Na+ form, 2 g), stirred for 10 minutes and filtered. To the filtrate was added ethanol (20 mL) and the solution was concentrated in vacuo to obtain the product (74 mg, 0.0864 mmols). ¹H NMR (500 MHz, CD₃OD) δ 1.70-1.72 (m, 2H), 1.95 (s, 3H), 3.02-3.06 (m, 2H), 3.10-3.27 (m, 4H), 4.25 (t, *J* = 6.4 Hz, 1H), 5.32 (s, 1H), 5.71 (s, 1H), 6.92 (d, *J* = 2.14 Hz, 2H), 7.02 (m, 2H), 7.20 (d, 2H, *J* = 9.5 Hz), 7.50 (d, *J* = 7.99 Hz, 1H), 8.14 (dd, *J₁* = 1.81 Hz, *J₂* = 7.96 Hz, 1H), 8.64 (d, *J* = 1.67 Hz, 1H).

### Sensor Preparation and Testing

The quencher, 4-(amidopropylmethacrylamide) N-benzyl (2-boronic acid) pyridinium bromide (23 mg), is dissolved in *N,N'*-dimethylacrylamide (100 mg) and *N,N'*-methylenebismethacrylamide (2 mg) and added to a solution of Sulforhodamine B CysMA (250 µL of a 2 mM aqueous solution), HCl (10 µL of a 1 M solution), 2,2'-azobis[2-(2-imidazolin-2-yl)propane] dihydrochloride (5 mg), and DI water (880 µL). A fraction of this solution is polymerized on the tip of a fiber optic sensor by heating at 37 °C for 24 h to form a hydrogel.

The sensor is tested by placing it in solutions containing different glucose concentrations ranging from 0 mg/dL to 400 mg/dL. The hydrogel indicator chemistry at the tip of the optical fiber is excited with light at a wavelength of 470 nm. Fluorescence emission is monitored between 550-700 nm.

While aspects of the present invention have been described in some detail for purposes of clarity and understanding, one skilled in the art will appreciate that various changes in form and detail can be made.

## Claims

1. An anionic, sulfonate-based fluorescent dye that is excited by visible light and emits at a wavelength greater than 500 nm wherein the dye is functionalized with at least one reactive group, said reactive group comprising an anionic group and an ethylenically unsaturated polymerizable group, wherein the said polymerizable group comprises aminoCysMA, with the proviso that said dye is of the non-pyrene type.

2. The functionalized dye of Claim 1, which emits at a wavelength greater than 550 nm.

3. The functionalized dye of Claim 1, which is excited by blue light having a wavelength of from 435 - 500 nm.

4. The functionalized dye of Claim 1, wherein said anionic group is selected from the group consisting of sulfonic acids, phosphonic acids, carboxylic acids, phenols, and their salts.

5. The functionalized dye of Claim 1, wherein said fluorescent dye is selected from the group consisting of sulforhodamine B, sulforhodamine 101, carboxytetramethylrhodamine, tetrakis(4-sulfophenyl)porphine, tetrakis(4-carboxy-phenyl)porphine, potassium perylenetetracarboxylate, Alexa Fluor 532, Alexa Fluor 546, Alexa Fluor 568, Alexa Fluor 594, BODIPY 576/589, BODIPY 581/591, BODIPY TR-X, SNARF-1, and Texas Red.

6. The compound according to claim 1 having the formula:
Dyeⁿ-R,
wherein Dyeⁿ-R is selected from the group consisting of: 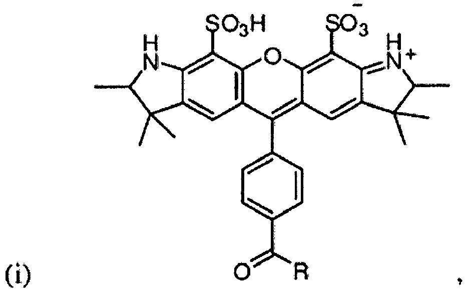 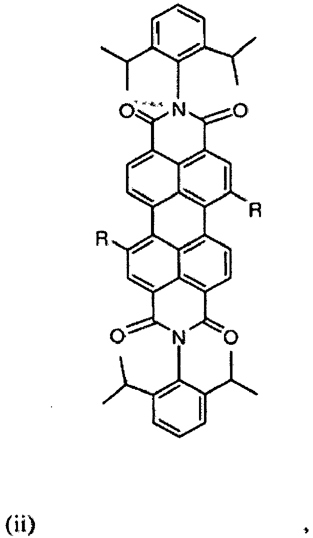 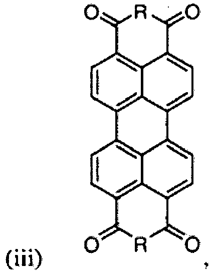 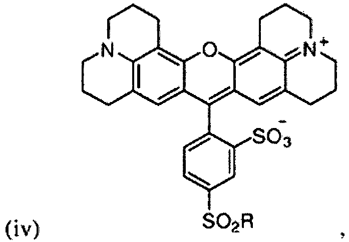 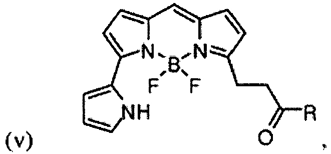 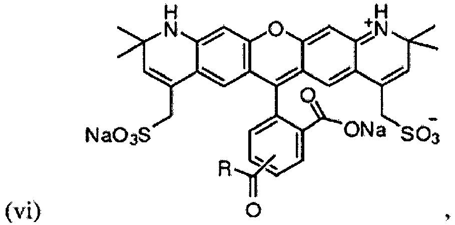 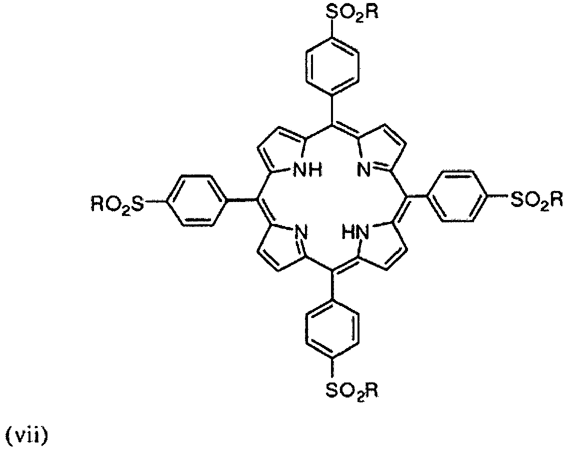 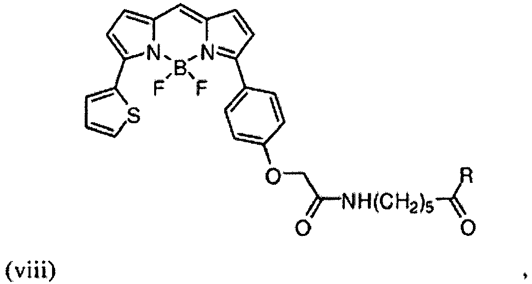 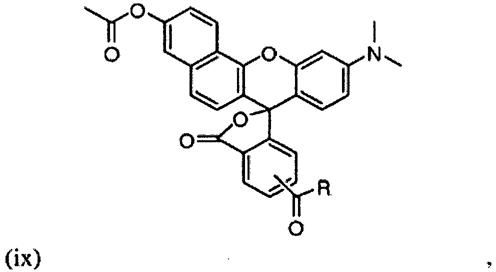 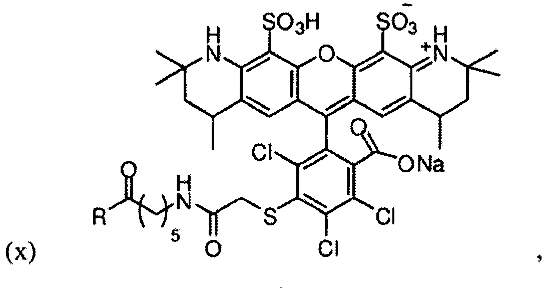 and 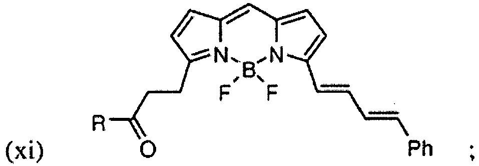 wherein: 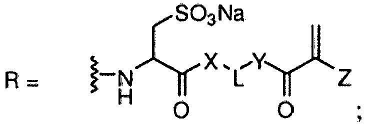 and wherein:
X is NH; Y is NH; L is -(CH₂)₃-
and Z is H or CH₃.

7. A method of making a dye according to Claim 6 comprising the step of: 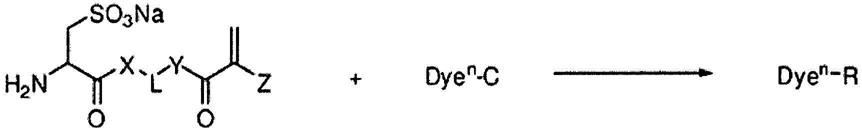 wherein Dyeⁿ-C is a reactive dye and C is a coupling group.

8. The compound: 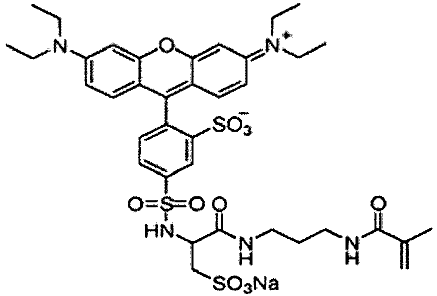 Sulforhodamine B CysMA (SBCMA).

9. A method of making the compound of Claim 8, comprising the step of:

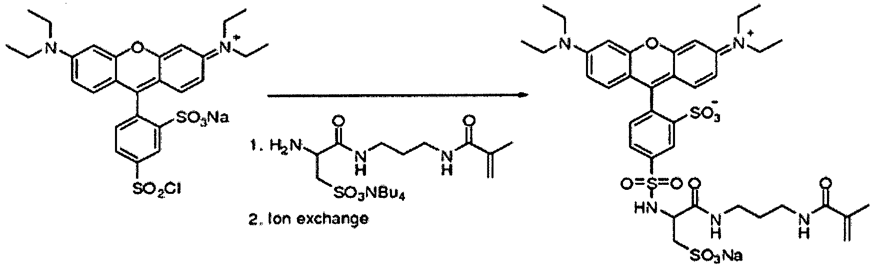

10. An analyte sensor for detecting a polyhydroxyl compound comprising an anionic, non-pyrene sulfonate-based fluorescent dye of Claim 1 and a pyridinium boronic acid quencher.

11. The analyte sensor of Claim 10, wherein said dye and said quencher are in the form of a polymer.

12. The analyte sensor of Claim 10, wherein said quencher comprises a monovalent, boronic acid- substituted pyridinium group.

13. The analyte sensor of Claim 10, wherein said quencher comprises a boronic acid-substituted polypyridinium, wherein said polypyridinium comprises two or more covalently coupled boronic acid-appended pyridinium groups.

14. A device for determining an analyte concentration comprising the analyte sensor of Claim 10.

15. The use of a fluorescence dye derivative as defined in claim 1 together with a pyridinium boronic acid quencher for the detection of a polyhydroxyl compound.

## Patentansprüche

1. Anionischer sulfonat-basierter Fluoreszenzfarbstoff, der durch sichtbares Licht angeregt wird und bei einer Wellenlänge größer als 500 nm emittiert, worin der Farbstoff mit mindestens einer reaktiven Gruppe funktionalisiert ist, besagte reaktive Gruppe eine anionische Gruppe und eine ethylenisch ungesättigt polymerisierbare Gruppe umfasst, worin die besagte polymerisierbare Gruppe aminoCysMA umfasst, mit der Bedingung, dass besagter Farbstoff vom Nicht-Pyren-Typ ist.

2. Der funktionalisierte Farbstoff nach Anspruch 1, der bei einer Wellenlänge größer als 550 nm emittiert.

3. Der funktionalisierte Farbstoff nach Anspruch 1, der durch blaues Licht mit einer Wellenlänge von 435 - 500 nm angeregt wird.

4. Der funktionalisierte Farbstoff nach Anspruch 1, worin besagte anionische Gruppe ausgewählt ist, aus der Gruppe bestehend aus Sulfonsäuren, Phosphonsäuren, Carboxylsäuren, Phenolen und deren Salze.

5. Der funktionalisierte Farbstoff nach Anspruch 1, worin besagter Fluoreszenzfarbstoff ausgewählt ist, aus der Gruppe bestehend aus Sulforhodamin B, Sulforhodamin 101, Carboxytetramethylrhodamin, Tetrakis(4-sulfophenyl)porphin, Tetrakis(4-carboxyphenyl)porphin, Kaliumperylentetracarboxylat, Alexa Fluor 532, Alexa Fluor 546, Alexa Fluor 568, Alexa Fluor 594, BODIPY 576/589, BODIPY 581/591, BODIPY TR-X, SNARF-1 und Texas Red.

6. Die Verbindung gemäß Anspruch 1 mit der Formel:
Dyeⁿ-R,
worin Dyeⁿ-R ausgewählt ist, aus der Gruppe bestehend aus: 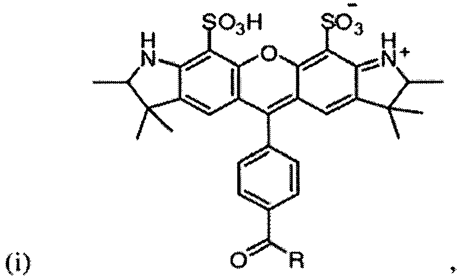 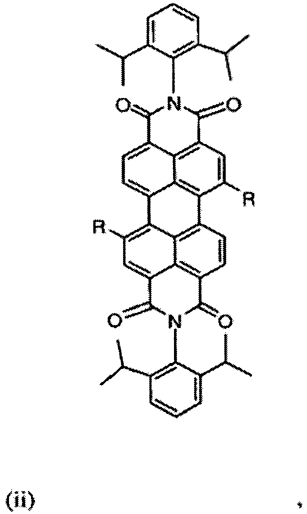 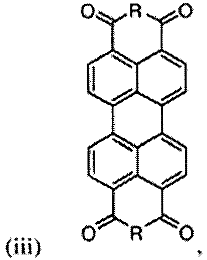 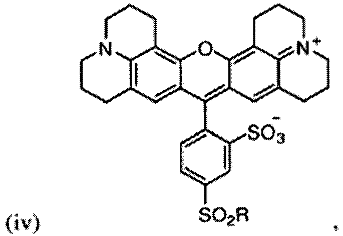 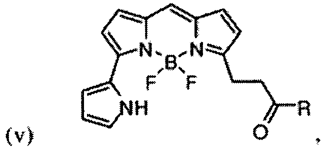 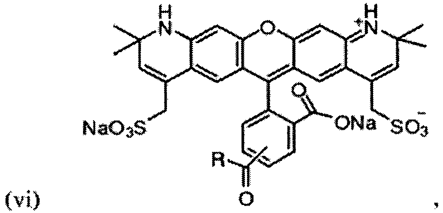 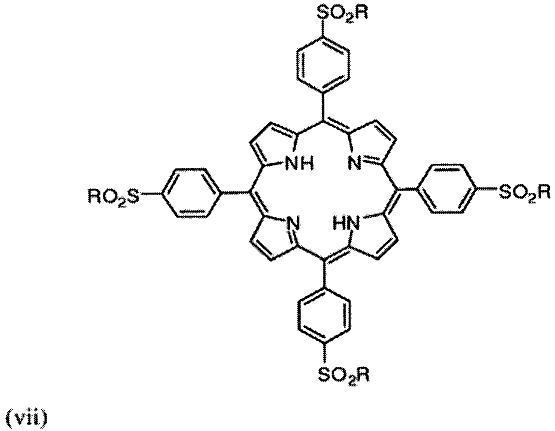 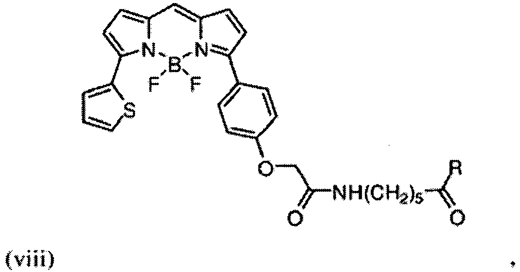 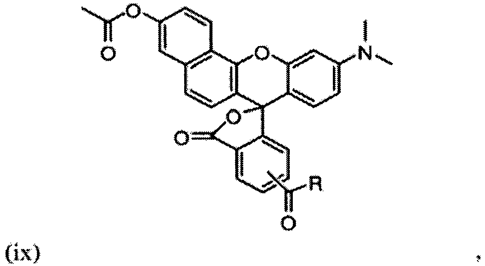 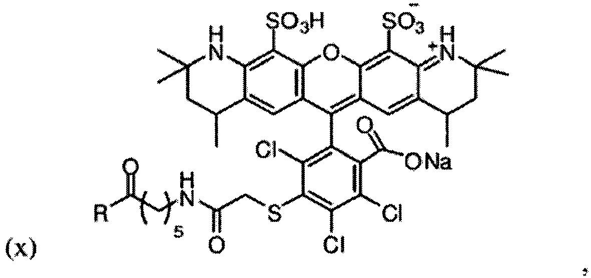 und 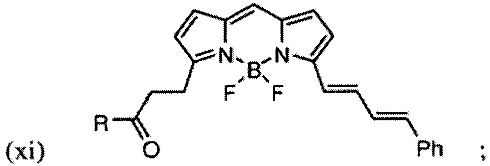 worin: 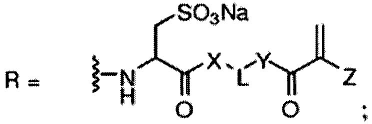 und worin:
X NH ist; Y NH ist; L -(CH₂)₃- ist
und Z H oder CH₃ ist.

7. Verfahren zur Herstellung eines Farbstoffs gemäß Anspruch 6 umfassend den Schritt: 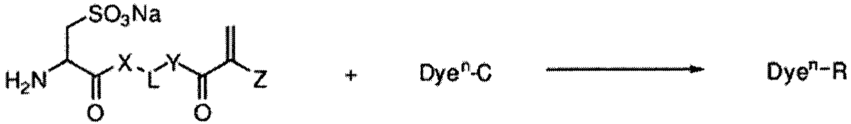 worin Dyeⁿ-C ein reaktiver Farbstoff und C eine Kopplungsgruppe ist.

8. Die Verbindung: 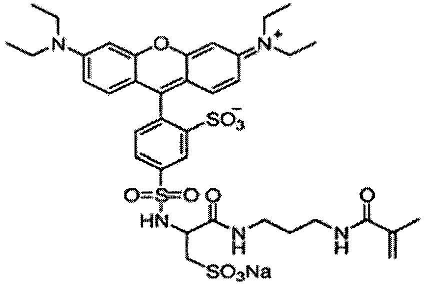 Sulforhodamine B CysMA (SBCMA).

9. Verfahren zur Herstellung der Verbindung nach Anspruch 8, umfassend den Schritt:

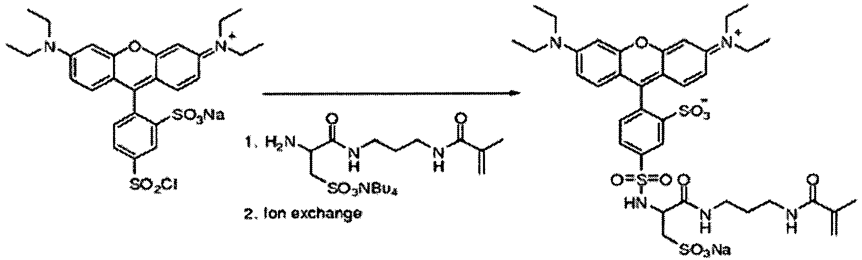

10. Analytsensor zum Nachweis einer Polyhydroxylverbindung umfassend einen anionischen, nicht-pyren sulfonat-basierten Fluoreszenzfarbstoff nach Anspruch 1 und einen Pyridiniumborsäure-Quencher.

11. Der Analytsensor nach Anspruch 10, worin besagter Farbstoff und besagter Quencher in Form eines Polymers vorliegen.

12. Der Analytsensor nach Anspruch 10, worin besagter Quencher eine monovalente, borsäuresubstituierte Pyridinium-Gruppe umfasst.

13. Der Analytsensor nach Anspruch 10, worin besagter Quencher ein borsäuresubstituiertes Polypyridinium umfasst, worin besagtes Polypyridinium zwei oder mehr kovalent gekoppelte Borsäure-anhängende Pyridinium-Gruppen umfasst.

14. Vorrichtung zur Bestimmung einer Analytkonzentration umfassend den Analytsensor nach Anspruch 10.

15. Verwendung eines Fluoreszenzfarbstoffderivates wie in Anspruch 1 definiert zusammen mit einem Pyridiniumborsäure-Quencher zum Nachweis einer Polyhydroxylverbindung.

## Revendications

1. Colorant fluorescent anionique à base de sulfonate qui est excité par la lumière visible et émet une longueur d'onde supérieure à 500 nm, le colorant étant fonctionnalisé avec au moins un groupe réactif, ledit groupe réactif comprenant un groupe anionique et un groupe polymérisable insaturé éthyléniquement, dans lequel ledit groupe polymérisable comprend un aminoCysMA, ledit colorant n'étant pas de type non-pyrène.

2. Colorant fonctionnalisé selon la revendication 1, qui émet à une longueur d'onde supérieure à 550 nm.

3. Colorant fonctionnalisé selon la revendication 1, qui est excité par une lumière bleue ayant une longueur d'onde de 435 à 500 nm.

4. Colorant fonctionnalisé selon la revendication 1, dans lequel ledit groupe anionique est choisi dans le groupe constitué d'acides sulfoniques, d'acides phosphoniques, d'acides carboxyliques, de phénols et de leurs sels.

5. Colorant fonctionnalisé selon la revendication 1, dans lequel ledit colorant fluorescent est choisi dans le groupe constitué de sulforhodamine B, sulforhodamine 101, carboxytétraméthylrhodamine, tétrakis(4-sulfophényl)porphine, tétrakis(4-carboxy-phényl)porphine, pérylènetétracarboxylate de potassium, Alexa Fluor 532, Alexa Fluor 546, Alexa Fluor 568, Alexa Fluor 594, BODIPY 576/589, BODIPY 581/591, BODIPY TR-X, SNARF-1 et Texas Red.

6. Composé selon la revendication 1 ayant la formule :
Colorantⁿ - R,
dans lequel Colorantⁿ - R est choisi dans le groupe constitué de : 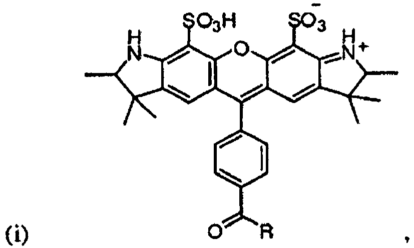 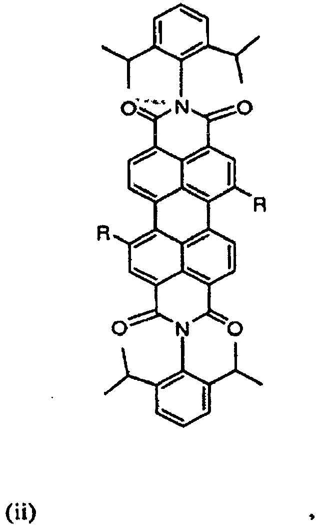 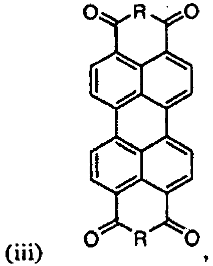 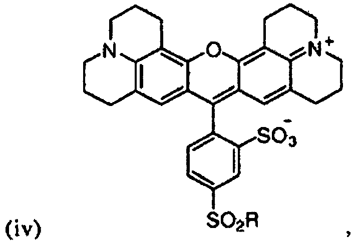 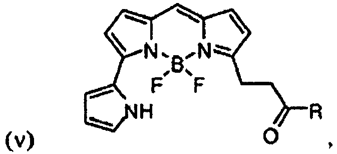 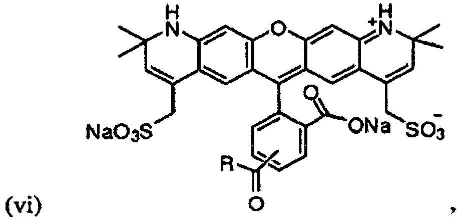 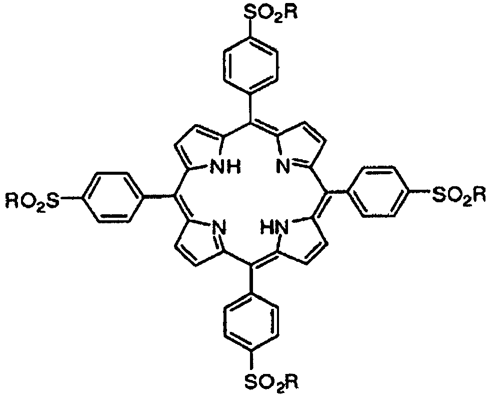 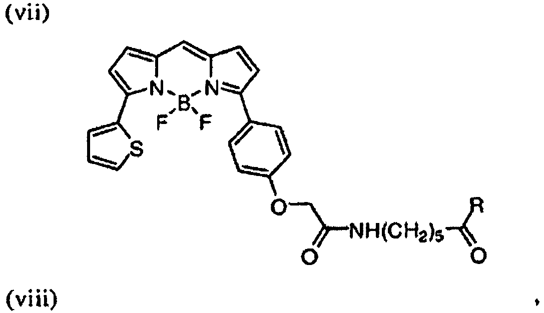 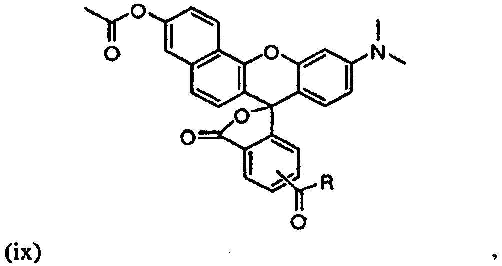 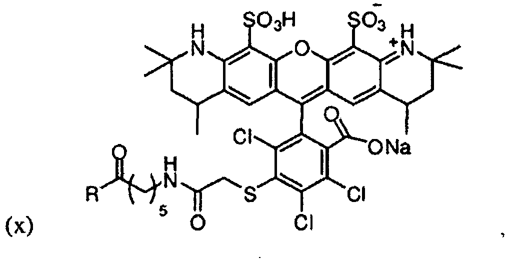 et 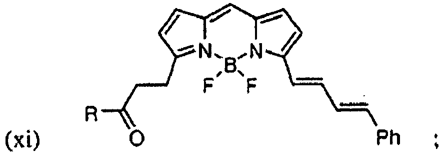 dans lequel : 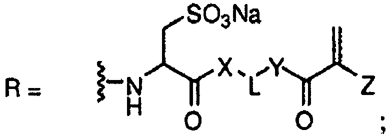 et dans lequel .
X est NH ; Y est NH ; L est -(CH₂)₃-
et Z est H ou CH₃.

7. Procédé de fabrication d'un colorant selon la revendication 6 comprenant l'étape suivante : 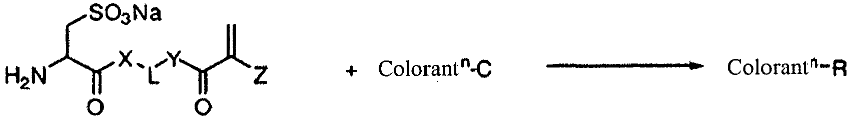 dans lequel Colorantⁿ - C est un colorant réactif et C est un groupe de couplage.

8. Composé : 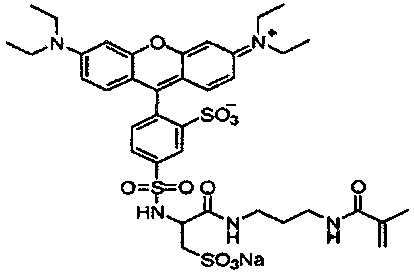 Sulforhodamine B CysMA (SBCMA).

9. Procédé de fabrication du composé selon la revendication 8, comprenant l'étape suivante :

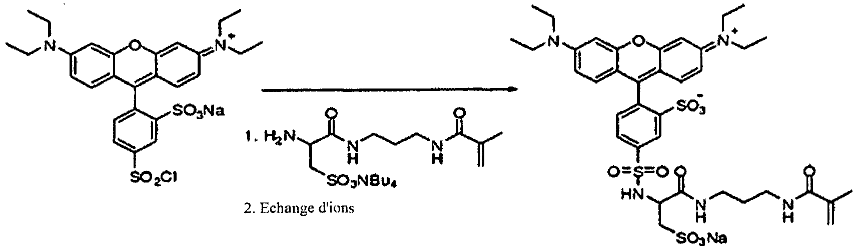

10. Capteur d'analyte pour détecter un composé polyhydroxyle comprenant un colorant fluorescent anionique à base de sulfonate non-pyrène selon la revendication 1 et un désactivateur de pyridinium-acide boronique.

11. Capteur d'analyte selon la revendication 10, dans lequel ledit colorant et ledit désactivateur sont sous la forme d'un polymère.

12. Capteur d'analyte selon la revendication 10, dans lequel ledit désactivateur comprend un groupe pyridinium monovalent substitué par acide boronique.

13. Capteur d'analyte selon la revendication 10, dans lequel ledit désactivateur comprend un polypyridinium substitué par acide boronique, dans lequel ledit polypyridinium comprend deux groupes pyridinium ou plus annexés à l'acide boronique couplés de manière covalente.

14. Dispositif pour déterminer une concentration d'analyte comprenant le capteur d'analyte selon la revendication 10.

15. Utilisation d'un dérivé de colorant fluorescent selon la revendication 1 en conjugaison avec un désactivateur de pyridinium-acide boronique pour la détection d'un composé polyhydroxyle.
